# EUROPEAN PATENT APPLICATION

(11) **EP 2 415 442 A1**
(43) Date of publication of application: **08.02.2012**
(21) Application number: 09842691.9
(22) Date of filing: 18.09.2009
(51) Int. Cl.: A61F 13/56, A61F 13/15, A61F 13/472

(54) **ABSORBENT ARTICLE**

(30) Priority: 31.03.2009 JP 2009087110
(71) Applicant: Unicharm Corporation, Ehime 799-0111 (JP)
(72) Inventor: NOMOTO, Takashi, Kanonji-shi Kagawa 769-1602 (JP); NAGATA, Akiyasu, Kanonji-shi Kagawa 769-1602 (JP)
(74) Representative: Peter, Julian
(86) International application number: PCT/JP2009/066872
(87) International publication number: WO 2010/113339

(57) **Abstract**

A sanitary napkin (1) comprises an absorbing body (2). The absorbing body (2) includes a fluid-permeable top sheet (3), a fluid-impermeable back sheet (4), and an absorber (5) arranged between the top sheet (3) and the back sheet (4). The napkin comprises a cover sheet (8) for covering the surface of the top sheet (3). A circumferential edge region (8P) of the cover sheet (8) is joined to the absorbing body (2). A flap for fixing the absorbing body (2) to underwear is sectionalized by a sectionalizing line (9) in the central region (8C) of the cover sheet. At the time of use, the flap is folded in a folding region and is expanded so as to be fixed to the underwear. A mark (20) is provided on the surface of the cover sheet (8) positioned substantially at the center of the absorbing body (2) to facilitate the positioning of the absorbing body (2) relative to clothing. The mark (20) is colored, but a surrounding portion (8S) surrounding the mark (20) is not colored.

## Description

### Technical Field

This invention relates to an absorbent article.

### Background Art

There has been known an absorbent article having an absorbing body. The absorbing body includes a fluid-permeable top sheet, a fluid-impermeable back sheet, and an absorber arranged between the top sheet and the back sheet. The top sheet and the back sheet are joined together on the outer region of the absorber. A graphic print is placed on the outer region on the user's body side of the back sheet or on the side of the top sheet (see PTL1). Namely, in this absorbent article, a printed layer is formed between the top sheet and the back sheet. The printed layer provides the emotional effect of reducing the depression of a user.

There has, further, been known an absorbent article having an absorbing body. The absorbing body includes a fluid-permeable top sheet, a fluid-impermeable back sheet, and an absorber arranged between the top sheet and the back sheet. A body fluid-permeable sheet is arranged between the top sheet and the absorber. The surface on the side of the absorber of the body fluid-permeable sheet is colored, or a pattern is printed on the side (see PTL2). Namely, in this absorbent article, a printed layer is formed on the back surface of the body fluid-permeable sheet under the top sheet. The printed layer is provided for improving the concealability of the body fluid that is absorbed.

Moreover, there has been known an absorbent article having an absorbing body. The absorbing body includes a fluid-permeable top sheet, a fluid-impermeable back sheet, and an absorber arranged between the top sheet and the back sheet. A plurality of compression grooves are provided extending in the longitudinal direction. The compression grooves are dented from the top sheet toward the back sheet (see PTL3). In this absorbent article, the compression grooves are provided so that the absorbing body undergoes deformation along the compression grooves so as to be more closely attached to the user's body.

### Citation List

### Patent Literature

PTL1 Japanese Patent No. 3974132
PTL2 Japanese Patent No. 3930741
PTL3 Japanese Patent No. 4180865

### Summary of Invention

Technical Problem

In order to prevent the leakage of fluid that is adsorbed, such as menses blood or urine, it is necessary that an absorbing body or an absorbent article is fixed to the underwear at a proper position. In particular in a dimly lit toilet, it is not necessarily easy to properly determine the position of the absorbing body relative to the underwear.

However, none of the above patent literatures address the above problem or disclose the means for solving the problem.

If the absorbing body is positioned relying on the printed layer or the compression grooves as taught by the above patent literatures, then it would seem the absorbing body can be easily fixed at a proper position.

However, with the absorbent article disclosed in PTL1, the printed layer is formed between the top sheet and the back sheet. Therefore, the printed layer may become illegible. With the absorbent article disclosed in PTL2, either, the printed layer is formed on the back surface of the body fluid-permeable sheet under the top sheet. Therefore, the printed layer may not be legible.

With the absorbent article disclosed in PTL 3, on the other hand, the top sheet must be exposed so that the compression grooves become legible, but this may invite a hygienic problem. In addition, the absorbing body is stiff around the compression grooves, and the user may feel it when the absorbing body comes in contact with the body of the user.

In any case, it is not easy to determine the position of the absorbing body.

### Solution to Problems

In order to solve the above problem, according to the present invention, there is provided an absorbent article comprising an absorbing body, the absorbing body including a fluid-permeable top sheet, a fluid-impermeable back sheet, and an absorber arranged between the top sheet and the back sheet, wherein the absorbent article further comprises a cover sheet for covering the surface of the top sheet, wherein a circumferential edge region of the cover sheet is joined to the absorbing body, and, in a central region of the cover sheet, at least one flap for fixing the absorbing body to clothing is sectionalized by a sectionalizing line which is constituted by a weakened line or a cut line, wherein, at the time of use, the flap is folded in a folding region and is expanded so as to be fixed to the clothing, and wherein a mark is provided on the cover sheet to facilitate the positioning of the absorbing body relative to the clothing.

### Advantageous Effect of Invention

The absorbing body can be easily fixed at a proper position of clothing.

### Brief Description of Drawings

Fig. 1 is a front view of a sanitary napkin.
Fig. 2 is a back view of the sanitary napkin.
Fig. 3 is a schematic transverse sectional view of the sanitary napkin along the line III-III in Fig. 1.
Fig. 4 is a front view of the sanitary napkin with the flaps being expanded.
Fig. 5 is a schematic longitudinal sectional view of the sanitary napkin illustrating the state of use.
Fig. 6 is a schematic transverse sectional view of the sanitary napkin illustrating the state of use.
Fig. 7 is a front view of the sanitary napkin according to another embodiment of the invention.
Fig. 8 is a front view of the sanitary napkin according to a further embodiment of the invention.
Figs. 9A and 9B are front views of the sanitary napkin showing alternative embodiments of the sectionalizing lines.
Fig. 10 is a front view of the sanitary napkin showing an alternative embodiment of the cover sheet.

### Description of Embodiments

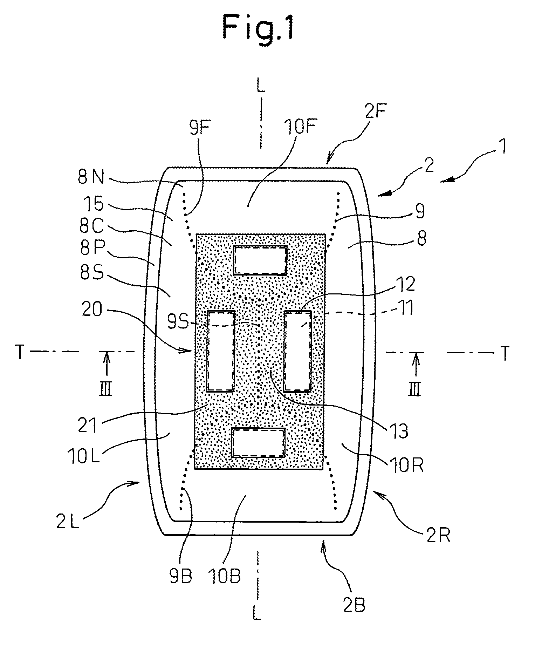

Figs. 1 to 3 illustrate a case where the invention is applied to a sanitary napkin. However, the invention can be, further, applied to other absorbent articles such as a panty liner, incontinence pad and the like.

Referring to Figs. 1 to 3, the sanitary napkin (hereinafter also referred to simply as "napkin") 1 according to the embodiment of the invention includes an absorbing body 2. The absorbing body 2 includes a fluid-permeable top sheet 3, a fluid-impermeable back sheet 4 and an absorber 5 capable of holding fluid being arranged between the top sheet 3 and the back sheet 4. The top sheet 3 and the back sheet 4 are substantially of the same size, and are joined together along the circumferential edges thereof by hot-melt adhesion or heat-sealing. Further, a sticking portion 6 is joined to the surface of the back sheet 4 that, in use, faces the clothing, such as underwear, so that the absorbing body 2 can be fixed to the underwear. The sticking portion 6 is covered with a protection sheet 7.

In Fig. 1, reference numerals 2F, 2B, 2L and 2R denote a front edge, a rear edge, a left side edge and a right side edge of the napkin 1 or the absorbing body 2. The front, rear, left and right are corresponding to the front, rear, left and right of the body of the user at the time of use.

The surface of the top sheet 3 that comes in contact with the user's skin at the time of use is covered with a fluid-impermeable cover sheet 8. The cover sheet 8 is substantially of the same size as the absorbing body 2, and therefore covers substantially the entire surface of the top sheet 3. Further, the cover sheet 8 is joined to the top sheet 3 or the absorbing body 2 along its annular circumferential edge region 8P by hot-melt adhesion or heat-sealing. On the other hand, a central region 8C other than the circumferential edge region 8P is not joined to the top sheet 3.

A sectionalizing line 9 such as a perforated weakened line is formed in advance in the central region 8C of the cover sheet 8, and a plurality of flaps are sectionalized or formed in the central region 8C by the sectionalizing line 9 to fix the absorbing body 2 to the underwear. The sectionalizing line 9 may be constituted by a cut line that completely cuts the cover sheet 8.

In the embodiment of the invention, the sectionalizing line 9 is constituted by a U-shaped curved portion 9F that expands toward the front edge 2F, a U-shaped curved portion 9B that expands toward the rear edge 2B, and a linear portion 9S that connects together the vertexes of the curved portions 9F and 9B substantially at the center of the cover sheet 8. Therefore, in the central region 8C, there are sectionalized four flaps, i.e., a semi-elliptical front flap 10F and rear flap 10B having curved ends, and a trapezoidal left flap 10L and right flap 10R. In this case, the front and rear flaps 10F and 10B are positioned symmetrically to each other, and the left and right flaps 10L and 10R are positioned symmetrically to each other, too.

The sectionalizing line 9, in this case, does not reach the circumferential edge region 8P, and therefore non-cut regions 8N are formed between the ends of the sectionalizing line 9 and the circumferential edge region 8P.

Sticking portions 11 are joined to the surfaces of these flaps 10F, 10B, 10L and 10R to fix the corresponding flaps 10F, 10B, 10L and 10R to the underwear. The sticking portions 11 are covered with separate protection sheets 12. These sticking portions 11 may be covered with a common protection sheet 12. In this case, the sticking portions 11 are joined to the flaps 10F, 10B, 10L and 10R being separated away from the sectionalizing line 9. Therefore, non-sticking regions 13 are formed on the flaps 10F, 10B, 10L and 10R surrounding the sticking portions 11.

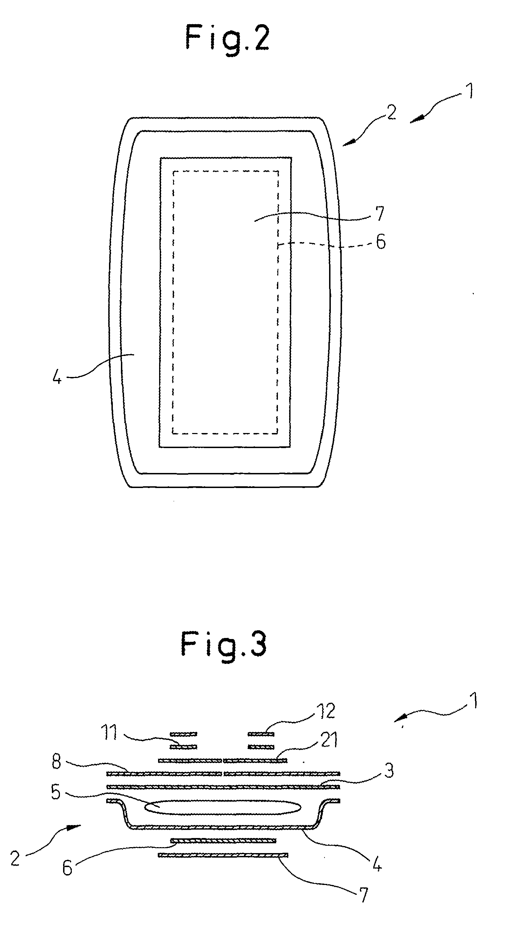

Further, a mark 20 is provided on the surface of the cover sheet 8 located substantially at the center of the absorbing body 2 to facilitate the positioning of the absorbing body 2 relative to the clothing, such as underwear. In the embodiment of the invention, the mark 20 is constituted by an additional sheet 21 that is joined by hot-melt adhesion or the like method to the surface of the cover sheet 8 located substantially at the center of the absorbing body 2. The additional sheet 20 has a rectangular shape with its major axis and minor axis extending on the longitudinal center line and the transverse center line of the absorbing body 2, respectively. The mark 20 may have any other shape, as a matter of course. As will be learned from Fig. 3, the sticking portions 11 are joined onto the additional sheet 21. The sectionalizing line 9 is formed after the additional sheet 21 is joined to the cover sheet 8, and therefore is also formed in the additional sheet 21.

A visual or tactile contrast is provided on the surface of the cover sheet 8 between the mark 20 and a surrounding portion 8S of the cover sheet 8 surrounding the mark 20. Namely, in the embodiment of the invention, the surface of the additional sheet 21 is colored; i.e., a character or a mark, a pattern or a color is printed thereon. On the other hand, no color is printed on the surrounding portion 8S. Further, a step is formed on the surface of the cover sheet 8 by a thickness of the additional sheet 21. The additional sheet 21 may be embossed.

Next, materials of the elements will be described.

The top sheet 3 is constituted by, for example, a porous or nonporous nonwoven fabric or a porous plastic sheet.

The back sheet 4 is constituted by, for example, a hydrophobic nonwoven fabric, a water-impermeable plastic film, a laminated sheet of a nonwoven fabric and a water-impermeable plastic film, a highly water resistant melt-blown nonwoven fabric, or an SMS nonwoven fabric sandwiched by spun-bonded nonwoven fabrics having a large strength.

The absorber 5 is constituted by, for example, a fluffy pulp or an air-laid nonwoven fabric and a highly absorbent polymer. The fluffy pulp is constituted by an artificial cellulose fiber such as chemical pulp, cellulose fiber, rayon or acetate, while the air-laid nonwoven fabric is constituted by a nonwoven fabric obtained by, for example, melt-adhering the pulp and the synthetic fiber together or by fixing them together with a binder, and the highly absorbent polymer is constituted by, for example, a granular or fibrous polymer of the type of starch, acrylic acid or amino acid.

The sticking portions 6 and 11 are constituted by a hot-melt adhesive such as styrene/isoprene/styrene block copolymer (SIS), styrene/butadiene/styrene block copolymer (SBS) or styrene/ethylene/butylene/ethylene copolymer (SEBS).

The cover sheet 8 is constituted by, for example, a hydrophobic nonwoven fabric, a water-impermeable plastic film, a laminated sheet of a nonwoven fabric and a water-impermeable plastic film, a highly water resistant melt-blown nonwoven fabric, or an SMS nonwoven fabric sandwiched by spun-bonded nonwoven fabrics having a large strength, and is, preferably, constituted by the hydrophobic nonwoven fabric. The basis weight of the cover sheet 8 is, preferably, from 15 g/m² to 60 g/m². When the cover sheet is constituted by using the plastic film, it is desired that the plastic film has a draping length of 20 mm to 100 mm and, more preferably, 30 mm to 70 mm relying on a cantilever method. If the draping length is shorter than 20 mm, the cover sheet 8 may be easily twisted. If the draping length is longer than 100 mm, on the other hand, the cover sheet 8 may become so hard as to deteriorate the feeling of use of the napkin 1 resulting in a decrease in the underwear follow-up performance and permitting the absorbed fluid to leak out.

The above cantilever method is conducted as described below in compliance with the JIS-L1018. Namely, five pieces of the object to be measured having a length of 150 mm and a width of 25 mm are overlapped one upon the other to obtain a measuring sample. Next, by using a cantilever manufactured by Daiei Kagaku Seiki Mfg. Co., the measuring sample is held under a holding plate of the cantilever and is slid in a tilted direction at a speed of 5 mm/sec to automatically measure a distance it has traveled. Measurements are taken in both of the cases of when the surface of the measuring sample faces down and when the back surface of the measuring sample is on the lower side, and an average value is regarded as the measured result.

The additional sheet 21 is constituted by, for example, a generally used nonwoven fabric such as air-through nonwoven fabric, heat-rolled nonwoven fabric, spun-bonded nonwoven fabric or melt-blown nonwoven fabric. However, it is, desired to use a nonwoven fabric produced by the air-through method since it offers thickness, hand and good touch feeling despite it is used in a low basis weight. The basis weight of the nonwoven fabric is desirably from 15 g/m² to 100 g/m². The material of the additional sheet 21 may be the same as the material of the cover sheet 8 but is, desirably, different.

The napkin 1 is fixed to the underwear as described below. Namely, the protection sheet 7 on the side of the back sheet 4 is removed, first. Next, the napkin 1 or the absorbing body 2 is positioned facing the inner surface of the underwear and is fixed to the underwear via the sticking portion 6.

Since the cover sheet 8 is provided with the mark 20, the user is allowed to properly position the absorbing body 2 easily and in a short period of time. The mark 20 provided on the surface of the cover sheet 8 is reliably legible by the user. Further, the top sheet 3 has been covered with the cover sheet 8 and is prevented from being fouled.

Next, the central region 8C of the cover sheet 8 is broken along the sectionalizing line 9. As a result, there are formed four flaps 10F, 10B, 10L and 10R that are separated from each other.

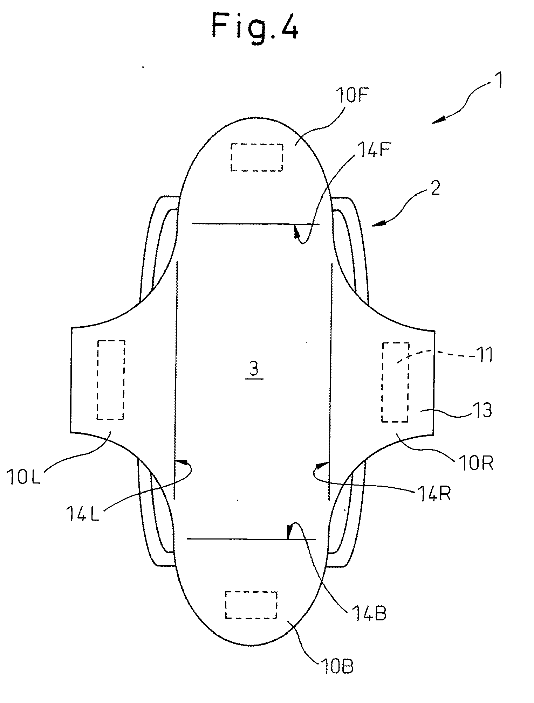

Next, as shown in Fig. 4, these flaps 10F, 10B, 10L and 10R are expanded, respectively. That is, the front flap 10F is expanded forward being folded in a folding region 14F along the front edge 2F, and the rear flap 10B is expanded rearward being folded in a folding region 14B along the rear edge 2B. Further, the left flap 10L is expanded toward the left being folded in a folding region 14L along the left edge 2L, and the right flap 10R is expanded toward the right being folded in a folding region 14R along the right edge 2R. As a result, the top sheet 3 is exposed.

According to the embodiment of the invention, as described above, the mark 20 is provided on the surface of the cover sheet 8. Therefore, there is no need of coloring the back surface of the cover sheet 8, i.e., there is no need of coloring the surface positioned on the user side when the flaps 10F, 10B, 10L and 10R are expanded, or the front surface of the top sheet 3. Therefore, no foul or offensive impression is given to the user.

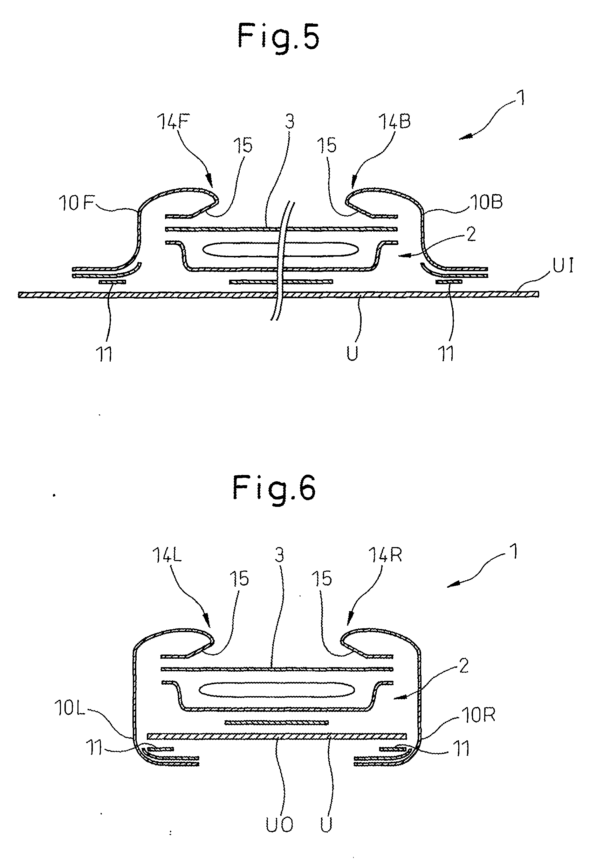

Next, the flaps 10F, 10B, 10L and 10R are attached to the underwear via the respective sticking portions 11. That is, as shown in Fig. 5 which is a longitudinal sectional view of the napkin 1, the front flap 10F is fixed to the inner surface UI of the underwear U in front of the absorbing body 2, and the rear flap 10B is fixed to the inner surface UI of the underwear U at the back of the absorbing body 2. As shown in Fig. 6 which is a transverse sectional view of the napkin 1, further, the left flap 10L and the right flap 10R are fixed to the outer surface UO of the underwear U under the absorbing body 2.

Thus, the flaps 10F, 10B, 10L and 10R are fixed to the underwear U and, therefore, the absorbing body 2 is fixed to the underwear U. In this case, the absorbing body 2 is fixed to the underwear U by both the flaps in the back-and-forth direction that are fixed expanded in the back-and-forth direction like the front flap 10F and the rear flap 10B, and the flaps in the right-and-left direction that are fixed expanded in the right-and-left direction like the left flap 10L and the right flap 10R. Further, the flaps fixed to the inner surface UI of the underwear like the front flap 10F and the rear flap 10B, are held sandwiched between the underwear U and the body of the user. On the other hand, the flaps fixed to the outer surface UO of the underwear U like the left flap 10L and the right flap 10R, work to hold the underwear U between these flaps and the absorbing body 2.

Despite external forces exerted on the absorbing body 2 from various directions, the absorbing body 2 can be suppressed from being twisted or deviated in position. Namely, the absorbing body 2 can be reliably fixed to the underwear U, and therefore the absorbed fluid is suppressed from leaking. In addition, the user is liberated from the anxiety of leakage.

According to the embodiment of the invention, the front ends of the flaps fixed to the inner surface like the front flap 10F and the rear flap 10B are curved, suppressing pain or offensive feeling that may be caused when the front flap 10F and the rear flap 10B come in contact with the body of the user.

Further, non-sticking regions 13 are formed on the flaps 10F, 10B, 10L and 10R or, concretely, at their end portions, enabling the user to handle the flaps 10F, 10B, 10L and 10R by picking up the non-sticking regions 13. It is, therefore, allowed to easily fix the absorbing body 2 to the underwear U.

As will be understood from Fig. 4, the folding regions 14F, 14B, 14L and 14R are positioned on straight lines connecting the two neighboring ends of the sectionalizing line 9. As shown in Fig. 1, on the other hand, non-cut regions 8N are formed between the ends of the sectionalizing line 9 and the circumferential edge region 8P. As shown in Figs. 5 and 6, therefore, the cover sheet 8 forms a leakage-preventing region 15 between the circumferential edge region 8P and the folded regions 14F, 14B, 14L and 14R or the flaps 10F, 10B, 10L and 10R, the leakage-preventing region 15 continuing over the whole circumference of the cover sheet 8. The leakage-preventing region 15 limits the fluid absorbed by the absorbing body 2 from flowing out and, therefore, works as a leakage-preventing wall along the surface of the top sheet 3.

In the embodiment of the invention, a piece of cover sheet 8 is simply joined to the absorbing body 2 to form the leakage-preventing region 15 that works as the leakage-preventing wall. Therefore, the leakage-preventing action can be easily obtained. The leakage-preventing region 15 has no seam. In addition, if the flaps 10F, 10B, 10L and 10R are pulled for fixing to the underwear U, the inner edge of the leakage-preventing region 15 rises from the top sheet 3, and the absorbed fluid is reliably trapped by the leakage-preventing region 15. This makes it possible to reliably suppress the leakage of the absorbed fluid.

According to the embodiment of the invention, further, the additional sheet 21 is joined to the flaps 10F, 10B, 10L and 10R surrounding the sticking portions 11, but is not joined to the surrounding portion 8S. In the flaps 10F, 10B, 10L and 10R, therefore, the regions surrounding the sticking portions 11 have a rigidity greater than the rigidity of other regions.

This makes it possible to easily handle the flaps 10F, 10B, 10L and 10R; i.e., the flaps 10F, 10B, 10L and 10R can be easily fixed to the underwear U.

This, in other words, means that the surrounding portion 8S inclusive of the folding regions 14F, 14B, 14L and 14R has a rigidity smaller than the rigidity of the mark 20. As a result, the flaps 10F, 10B, 10L and 10R can be easily folded and expanded.

As shown in Fig. 1, further, the mark 20 includes portions of the flaps 10F, 10B, 10L and 10R. That is, portions or ends of the flaps 10F, 10B, 10L and 10R are constituted by the mark 20, and the rests of them are constituted by the surrounding portion 8S. Therefore, the flaps 10F, 10B, 10L and 10R and, particularly, the ends thereof are reliably legible by the user; i.e., the flaps 10F, 10B, 10L and 10R can be easily picked up and expanded.

The width of the circumferential edge region 8P is, desirably, 2 mm to 10 mm and, more desirably, 3 mm to 5 mm. This is because if the width of the circumferential edge region 8P is smaller than 2 mm, then the junction strength of the cover sheet 8 and the absorbing body 2 decreases. If the width of the circumferential edge region 8P is larger than 10 mm, the exposed surface of the top sheet 3 becomes small when the flaps 10F, 10B, 10L and 10R are expanded. Namely, if the top sheet is broadly exposed, leakage of the absorbed fluid can be suppressed even in case the position where the napkin 1 or the absorbing body 2 is fixed to the underwear U is deviated from the proper position to some extent.

On the other hand, the width of the non-cut regions 8N is, desirably, 2 mm to 10 mm and, more desirably, 3 mm to 5 mm. This is because if the width of the non-cut regions 8N is smaller than 2 mm, the tensile strength of the cover sheet 8 in the non-cut regions 8N decreases and, therefore, the leakage-preventing effect of the leakage-preventing region 15 decreases. If the width of the non-cut regions 8N is larger than 10 mm, the exposed surface of the top sheet 3 becomes small when the flaps 10F, 10B, 10L and 10R are expanded.

The ratio of the area of the mark 20 to the area of the cover sheet 8 is, desirably, 10% to 90% and, more desirably, 40% to 70%. If the ratio becomes smaller than 10% or becomes larger than 90%, the visual or tactile contrast decreases between the mark 20 and the circumferential portion 8S, and the mark 20 becomes less legible.

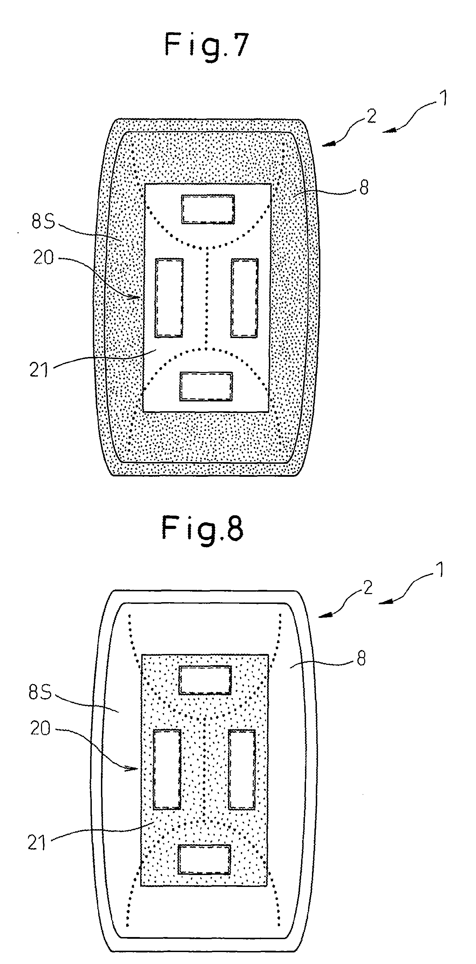

In the foregoing description, the additional sheet 21 is colored but the surrounding portion 8S is not colored. However, as shown in Fig. 7, the surrounding portion 8S may be colored without coloring the additional sheet 21.

As shown in Fig. 8, the additional sheet 21 may be embossed without embossing the surrounding portion 8S. Conversely, the surrounding portion 8S may be embossed without embossing the additional sheet 21.

Moreover, the additional sheet 21 may not be provided, and the surface of the cover sheet 8 positioned substantially at the center of the absorbing body 2 may be directly colored or embossed.

Generally speaking, therefore, either the mark 20 or the surrounding portion 8S is colored or embossed and the other one is neither colored nor embossed.

Both the mark 20 and the surrounding portion 8S may be colored or embossed to attain a visual or tactile contrast.

In the embodiment shown in Fig. 8, if the embossing is a rugged pattern-imparting working, the contact area can be decreased between the flaps 10F, 10B and the body of the user, preventing stuffiness and improving touch feeling. This, further, excels in transferring the hot-melt adhesive of when the sticking portions 11 are joined to the flaps 10F, 10B, 10L and 10R.

In the above description, further, the mark 20 is provided on the surface of the cover sheet 8 positioned substantially at the center of the absorbing body 2. However, the mark 20 may be provided at a position other than the center of the absorbing body 2 or on the back surface of the cover sheet 8 so far as the user finds it easy to locate the absorbing body 2 at a desired position.

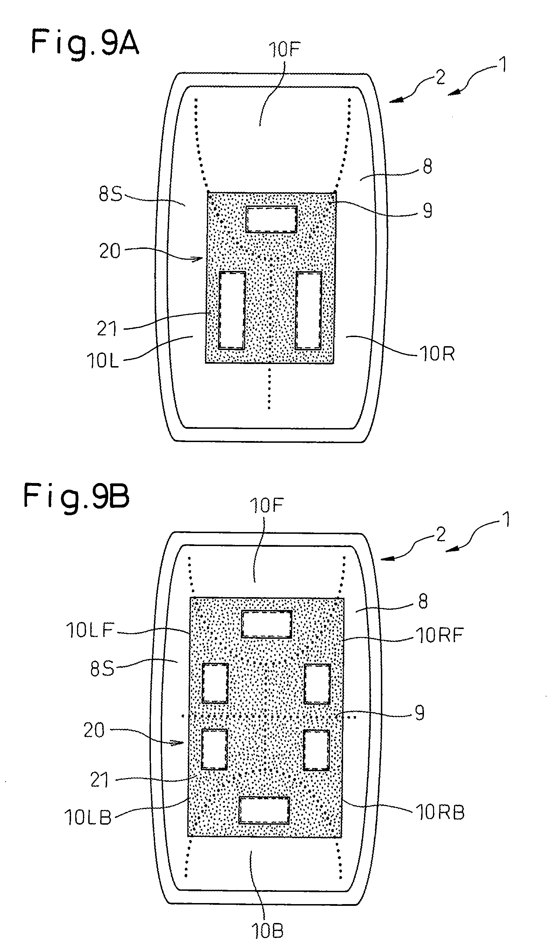

In the foregoing description, further, the sectionalizing line 9 is so formed that four flaps are sectionalized in the central region 8C of the cover sheet 8. However, the number of the flaps may not have to be four. That is, as shown in Fig. 9A, the sectionalizing line 9 may be so formed as to sectionalize three flaps 10F, 10L and 10R in the central region 8C. Or, as shown in Fig. 9B, the sectionalizing line 9 may be so formed as to sectionalize six flaps 10F, 10B, 10LF, 10LB, 10RF and 10RB in the central region 8C.

Generally speaking, therefore, at least one flap is sectionalized by the sectionalizing line 9 in the central region 8C of the cover sheet 8.

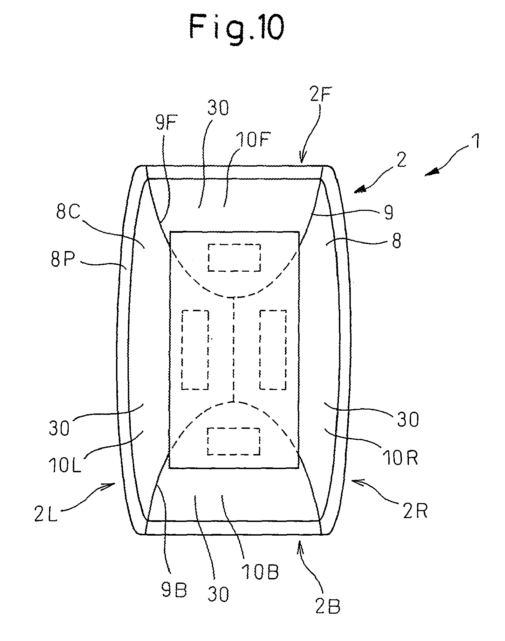

In the foregoing description, further, the cover sheet 8 is constituted by a piece of sheet. However the cover sheet 8, can be constituted by a plurality of pieces of separate cover sheet portions. Namely, in an embodiment shown in Fig. 10, the coversheet 8 is constituted by, for example, four pieces of cover sheet portions 30. In this case, the cover sheet portions 30 cover portions of the surface of the top sheet 3; i.e., the cover sheet portions 30 substantially cover the entire surface of the top sheet 3. Further, the sectionalizing lines 9 formed among the neighboring cover sheet portions 30 are extending traversing the circumferential edge region 8P. In the embodiment shown in Fig. 10, further, the cover sheet portions 30 are forming the flaps 10F, 10B, 10L and 10R, respectively. However, the plurality of flaps 10F, 10B, 10L and 10R may be formed by a piece of cover sheet portion 30. Further, the cover sheet portions 30 may be overlapped one upon the other.

### Reference Signs List

- 1: sanitary napkin
- 2: absorbing body
- 3: top sheet
- 4: back sheet
- 5: absorber
- 8: cover sheet
- 8C: central region
- 8P: circumferential edge region
- 8S: surrounding portion
- 9: sectionalizing line
- 10F: front flap
- 10B: rear flap
- 10L: left flap
- 10R: right flap
- 11: sticking portions
- 14F, 14B, 14L, 14R: folding regions
- 20: mark
- 21: additional sheet

## Claims

1. An absorbent article comprising an absorbing body, the absorbing body including a fluid-permeable top sheet, a fluid-impermeable back sheet, and an absorber arranged between the top sheet and the back sheet,
wherein the absorbent article further comprises a cover sheet for covering the surface of the top sheet,
wherein a circumferential edge region of the cover sheet is joined to the absorbing body, and, in a central region of the cover sheet, at least one flap for fixing the absorbing body to clothing is sectionalized by a sectionalizing line which is constituted by a weakened line or a cut line,
wherein, at the time of use, the flap is folded in a folding region and is expanded so as to be fixed to the clothing, and
wherein a mark is provided on the cover sheet to facilitate the positioning of the absorbing body relative to the clothing.

2. The absorbent article according to claim 1, wherein a visual or tactile contrast is provided between the mark and a surrounding portion which is a surface portion of the cover sheet surrounding the mark.

3. The absorbent article according to claim 1, wherein the mark is constituted by an additional sheet joined to the cover sheet.

4. The absorbent article according to claim 1, wherein the mark forms a part of the flap.

5. The absorbent article according to claim 1, wherein the flap includes a front flap and a rear flap that are folded in the folding regions along the front and rear edges of the absorbing body and are expanded in the back-and-forth direction, and a left flap and a right flap that are folded in the folding regions along the left and right side edges of the absorbing body and are expanded in the right-and-left direction.

6. The absorbent article according to claim 1, wherein the mark is of a rectangular shape having a major axis extending on a longitudinal center line of the absorbing body and a minor axis extending on a transverse center line of the absorbing body.

7. The absorbent article according to claim 1, wherein the cover sheet is fluid-impermeable.
